# EUROPEAN PATENT APPLICATION

(11) **EP 3 884 920 A1**
(43) Date of publication of application: **29.09.2021**
(21) Application number: 19886511.5
(22) Date of filing: 19.11.2019
(51) Int. Cl.: A61K 6/00

(54) **DENTAL CLEANING MATERIAL AND DENTAL TEMPORARY ADHESIVE MATERIAL REMOVAL MATERIAL**

(30) Priority: 20.11.2018 JP 2018217303; 21.11.2018 JP 2018218485; 28.06.2019 JP 2019122136
(71) Applicant: Kuraray Noritake Dental Inc., Kurashiki-shi, Okayama 710-0801 (JP)
(72) Inventor: OKADA Keishu, Tainai-shi, Niigata 959-2653 (JP); KAWANA Mariko, Tainai-shi, Niigata 959-2653 (JP); KASHIKI Nobusuke, Tainai-shi, Niigata 959-2653 (JP); NOJIRI Yamato, Tokyo 100-0004 (JP); TAJIRI Yuko, Suita-shi, Osaka 565-0871 (JP); MINE Atsushi, Suita-shi, Osaka 565-0871 (JP); YATANI Hirofumi, Suita-shi, Osaka 565-0871 (JP)
(74) Representative: D Young & Co LLP
(86) International application number: PCT/JP2019/045299
(87) International publication number: WO 2020/105642

(57) **Abstract**

The present invention provides a dental cleaner that provides desirable cleanliness against contamination of dental restorations and abutment teeth due to protein-containing body fluids while ensuring uniform quality by reducing precipitation of salts, and that ensures desirable bond durability after cleaning while being safe to use in the oral cavity. The present invention relates to a dental cleaner comprising an acidic group-containing radical polymerizable monomer (a), water (b), and an organic amine compound (c). The present invention also relates to a dental remover for temporary adhesives comprising an acidic group-containing compound (A), water (B), and a basic compound (C).

## Description

### TECHNICAL FIELD

The present invention relates to a dental cleaner used for cleaning of dental restorations such as porcelains, ceramics, metal oxides, and cured products of dental composite resins containing inorganic filler and resin (hereinafter, simply "cured products of dental composite resins"), and for cleaning of an abutment tooth such as a tooth surface. The present invention also relates to a dental remover for temporary adhesives.

### BACKGROUND ART

In dental restorative treatment, dental restorative materials such as porcelains, ceramics, metal oxides, and cured products of dental composite resins are fitted over missing parts of teeth in a variety of forms. Fixation of such dental restorative materials is typically preceded by a trial procedure called try-in, in which a dental restoration is tried in to confirm fit. In practice, the procedure unavoidably involves contamination of dental restorations and abutment teeth by protein-containing body fluids (for example, saliva, dentinal fluid, blood), even when a dental rubber dam is used. In subsequent bonding of a dental restoration and an abutment tooth, such protein-containing body fluids weaken the adhesive properties between the dental restoration and a dental bonding agent or a dental cement, or between the abutment tooth and a dental bonding agent or a dental cement. Dental cleaners are proposed as a means to remove contamination due to protein-containing body fluids (Patent Literatures 1 and 2).

Patent Literature 1 describes a tooth surface cleaner as a way of removing plaque and color on tooth surface. Patent Literature 2 describes a composition containing cleaning particles for cleaning dental restorations, particularly for removal of contaminant protein.

In temporary restoration of large cavities and abutment teeth where greater importance is placed on retention of occlusion and contacts, temporary restorations fabricated from materials such as autopolymerizing resins are often temporarily placed using a temporary material called temporary adhesive. In treatments that do not use a prosthesis as a final restoration such as in restorative filling treatment, materials used for this purpose are often called temporary sealants. However, in this patent specification, such temporary sealants are also regard as a type of temporary adhesive.

Unlike luting materials used for luting of a final restoration, temporary adhesives do not need to sustain high bond strength over prolonged time periods but require the bond strength high enough to hold a temporary restoration on a cavity or abutment tooth for a relatively short time period of one to several weeks before the final restoration is placed. Typical examples of temporary adhesives include (1) Fletcher's cements, (2) eugenol-containing temporary adhesives, (3) eugenol-free temporary adhesives, and (4) carboxylic acid-based temporary adhesives (see, for example, Patent Literature 3).

(1) Fletcher's cements contain zinc oxide and gum arabic as main components, and are used after mixing these components into slurry form using distilled water. (2) Eugenol-containing temporary adhesives typically contain zinc oxide and eugenol as main components, and many of eugenol-containing temporary adhesives are available as temporary sealants. (3) Eugenol-free temporary adhesives contain zinc oxide and aliphatic or aromatic compounds as main components, without eugenol. (4) Carboxylic acid-based temporary adhesives contain zinc oxide and polyacrylic acid as main components, together with a tannin-fluoride mixture (HY agent) having the effect to strengthen tooth structure (dentin).

Desirably, a temporary adhesive should be completely removed from tooth structures such as tooth cavities and abutment teeth before luting of a final restoration. Any remaining temporary adhesive is known to seriously affect the bond strength between the final restoration and tooth structure (see, for example, Non Patent Literature 1).

A mechanical process using an air scaler or a rotary brush, and ultrasonic cleaning are examples of methods currently available for removal of temporary adhesives (see, for example, Non Patent Literature 2).

### CITATION LIST

### Patent Literature

Patent Literature 1: JP 2016-003213 A
Patent Literature 2: Japanese Patent No. 5607559
Patent Literature 3: Japanese Patent No. 5522909

### Non Patent Literature

Non Patent Literature 1: Journal of Dentistry, Volume 40, Issue 2, February 2012, Pages 131-138.
Non Patent Literature 2: Journal of Oral Science 2005 ; Vol.47, No.1, 9-13.

### SUMMARY OF INVENTION

### Technical Problem

Patent Literature 1 describes a dental cleaner containing water of pH 12 to 13, a surfactant, and a basic substance. However, because of high alkalinity, the dental cleaner disclosed in this related art document cannot be used in the oral cavity, and poses a safety issue. Patent Literature 2 describes a composition containing cleaning particles for removal of contaminant protein, in the context of fixing dental restoration materials with an adhesive. However, the composition disclosed in this related art document is also problematic in terms of safety because the composition is a basic substance containing sodium hydroxide, and cannot be used in the oral cavity.

A dental cleaner must restrain from generating precipitates during storage in a refrigerator. Formation of precipitates makes the composition nonuniform, and poses a possibility of creating variations in quality. The solid also has a risk of weakening adhesive properties when it remains unremoved from prosthesis surface after cleaning.

Non Patent Literatures 1 and 2 discuss a possible decrease of bond strength when clumps of temporary adhesive are removed with an air scaler or when sonication is used for cleaning. Non Patent Literature 2 describes removing a temporary adhesive by the mechanical stimulation of cleaning with a rotary brush. However, this removal method is highly dependent on the skills of the operator, and not all clinics are equipped with the instruments needed to carry out this process. These cleaning methods are therefore not always recommendable, and there is a need for a method that enables easier removal of temporary adhesives.

Under these circumstances, an object of the present invention is to provide a dental cleaner that provides desirable cleanliness against contamination of dental restorations and abutment teeth due to protein-containing body fluids while ensuring uniform quality by reducing generation of precipitates, and that ensures desirable bond durability after cleaning while being safe to use in the oral cavity. Against the backdrop of the related art discussed above, another object of the present invention is to provide a dental remover for temporary adhesives that conveniently provides desirable cleanliness against temporary adhesives without a mechanical process while ensuring desirable bond strength after cleaning.

### Solution to Problem

The present inventors conducted intensive studies, and found that the foregoing object concerning a dental cleaner can be achieved by a dental cleaner that comprises an acidic group-containing radical polymerizable monomer (a), water (b), and an organic amine compound (c). The present inventors also found that the foregoing object concerning a dental remover for temporary adhesives can be achieved by a dental remover for temporary adhesives that comprises an acidic group-containing compound (A), water (B), and a basic compound (C). The present invention was completed on the basis of these findings. Specifically, the present invention includes the following.
[1] A dental cleaner comprising an acidic group-containing radical polymerizable monomer (a), water (b), and an organic amine compound (c).
[2] The dental cleaner according to [1], wherein the dental cleaner has a pH of less than 8.0.
[3] The dental cleaner according to [1] or [2], wherein the acidic group-containing radical polymerizable monomer (a) comprises at least one monomer selected from the group consisting of a phosphoric acid group-containing (meth)acrylic monomer, a pyrophosphoric acid group-containing (meth)acrylic monomer, and a carboxylic acid group-containing (meth)acrylic monomer.
[4] The dental cleaner according to any one of [1] to [3], wherein the organic amine compound (c) comprises a tertiary organic amine compound.
[5] The dental cleaner according to [4], wherein the tertiary organic amine compound comprises an aliphatic compound.
[6] The dental cleaner according to [5], wherein the aliphatic compound is triethanolamine and/or 2-(dimethylamino)ethyl methacrylate.
[7] The dental cleaner according to any one of [4] to [6], wherein the tertiary organic amine compound comprises an aromatic compound.
[8] The dental cleaner according to any one of [1] to [7], wherein the dental cleaner further comprises a basic inorganic compound (d).
[9] The dental cleaner according to [8], wherein the basic inorganic compound (d) is at least one basic substance selected from the group consisting of a phosphate, a hydrogen phosphate, and a dihydrogen phosphate.
[10] The dental cleaner according to [8] or [9], wherein the basic inorganic compound (d) is at least one selected from the group consisting of an alkali metal salt of phosphoric acid, an alkali metal salt of hydrogen phosphate, and an alkali metal salt of dihydrogen phosphate.
[11] The dental cleaner according to any one of [1] to [10], wherein the dental cleaner further comprises at least one gelatinizer (e) selected from the group consisting of a hydrophilic filler (e-1), a hydrophobic filler (e-2), and a polymeric thickener (e-3).
[12] The dental cleaner according to [11], wherein the gelatinizer (e) is a hydrophilic filler (e-1) and a hydrophobic filler (e-2).
[13] The dental cleaner according to [12], wherein the hydrophilic filler (e-1) is hydrophilic fumed silica, and the hydrophobic filler (e-2) is hydrophobic fumed silica.
[14] The dental cleaner according to [12] or [13], wherein the hydrophilic filler (e-1) and the hydrophobic filler (e-2) have a mass ratio of 4:6 to 2:8.
[15] A method for producing a dental cleaner, comprising mixing an acidic group-containing radical polymerizable monomer (a), water (b), and an organic amine compound (c).
[16] Nontherapeutic use of a dental cleaner comprising an acidic group-containing radical polymerizable monomer (a), water (b), and an organic amine compound (c) for removal of a dental temporary adhesive.
[17] A dental remover for temporary adhesives, comprising an acidic group-containing compound (A), water (B), and a basic compound (C).
[18] The dental remover for temporary adhesives according to [17], wherein the dental remover has a pH of less than 8.0.
[19] The dental remover for temporary adhesives according to [17] or [18], wherein the acidic group-containing compound (A) is an acidic group-containing polymerizable monomer (A-1).
[20] The dental remover for temporary adhesives according to [19], wherein the acidic group-containing polymerizable monomer (A-1) is at least one selected from the group consisting of a phosphoric acid group-containing (meth)acrylic monomer, a pyrophosphoric acid group-containing (meth)acrylic monomer, and a carboxylic acid group-containing (meth)acrylic monomer.
[21] The dental remover for temporary adhesives according to any one of [17] to [20], wherein the basic compound (C) is at least one selected from the group consisting of a basic inorganic compound (C-1) and an organic amine compound (C-2).
[22] The dental remover for temporary adhesives according to [21], wherein the basic inorganic compound (C-1) is at least one basic substance selected from the group consisting of a phosphate, a hydrogen phosphate, and a dihydrogen phosphate.
[23] The dental remover for temporary adhesives according to [21] or [22], wherein the basic inorganic compound (C-1) is at least one selected from the group consisting of an alkali metal salt of phosphoric acid, an alkali metal salt of hydrogen phosphate, and an alkali metal salt of dihydrogen phosphate.
[24] The dental remover for temporary adhesives according to [21], wherein the organic amine compound (C-2) comprises a tertiary organic amine compound.
[25] The dental remover for temporary adhesives according to any one of [17] to [24], wherein the dental remover further comprises a gelatinizer (D).
[26] The dental remover for temporary adhesives according to [25], wherein the gelatinizer (D) comprises a hydrophilic filler (D-1) and a hydrophobic filler (D-2).
[27] The dental remover for temporary adhesives according to [26], wherein the hydrophilic filler (D-1) is hydrophilic fumed silica, and the hydrophobic filler (D-2) is hydrophobic fumed silica.
[28] The dental remover for temporary adhesives according to [26] or [27], wherein the hydrophilic filler (D-1) and the hydrophobic filler (D-2) have a mass ratio of 4:6 to 2:8.
[29] The dental remover for temporary adhesives according to any one of [25] to [28], wherein the gelatinizer (D) comprises a polymeric thickener (D-3).
[30] A method for producing a dental remover for temporary adhesives of [17], comprising mixing an acidic group-containing compound (A), water (B), and a basic compound (C).

### Advantageous Effects of Invention

A dental cleaner of the present invention provides desirable cleanliness against contamination of dental restorations and abutment teeth due to protein-containing body fluids, and ensures desirable bond durability after cleaning while being safe to use in the oral cavity. Because a dental cleaner of the present invention does not generate solid precipitates during storage or use, there is no risk of creating variations in quality, or interfering with bonding. A dental cleaner of the present invention also has desirable antiseptic properties, preventing growth of microorganisms such as fungi. A dental remover for temporary adhesives of the present invention provides desirable cleanliness against temporary adhesives-a material used temporarily only for a short duration of time-while ensuring desirable bond strength after cleaning.

### DESCRIPTION OF EMBODIMENTS

A feature of a dental cleaner (a dental cleaner composition) of the present invention is that it comprises an acidic group-containing radical polymerizable monomer (a), water (b), and an organic amine compound (c).

The following describes the acidic group-containing radical polymerizable monomer (a), water (b), and organic amine compound (c) constituting a dental cleaner of the present invention.

The acidic group-containing radical polymerizable monomer (a) used in the present invention is described first.

The acidic group-containing radical polymerizable monomer (a) reacts with organic amine compound (c) to form salts, and, through surfactant action, improves the cleaning effect against contamination caused by protein-containing body fluids or by substances originating from such bodily fluids (for example, plaque, pellicle). The acidic group-containing radical polymerizable monomer (a) penetrates and binds to tooth structure through demineralization, and provides improved adhesive properties for tooth structure. By combining with organic amine compound (c), the acidic group-containing radical polymerizable monomer (a) also provides desirable bond durability for tooth structure and dental prostheses after cleaning. The acidic group-containing radical polymerizable monomer (a), by combining with organic amine compound (c), exhibits antiseptic properties, and prevents growth of microorganisms such as fungi. The acidic group-containing radical polymerizable monomer (a) is a polymerizable monomer having at least one acidic group such as a phosphoric acid group, a phosphonic acid group, a pyrophosphoric acid group, a carboxylic acid group, and a sulfonic acid group, and at least one radical polymerizable group such as an acryloyl group, a methacryloyl group, an acrylamide group, and a methacrylamide group. Preferably, the acidic group-containing radical polymerizable monomer (a) is at least one selected from the group consisting of a phosphoric acid group-containing (meth)acryhc monomer, a pyrophosphoric acid group-containing (meth)acrylic monomer, and a carboxylic acid group-containing (meth)acrylic monomer. In view of adhesive properties for enamel, the acidic group-containing radical polymerizable monomer (a) is preferably a monofunctional acidic group-containing (meth)acrylic monomer having any one of an acryloyl group, a methacryloyl group, an acrylamide group, and a methacrylamide group. Specific examples include the following. As used herein, "methacryl" and "acryl" are collectively referred to as "(meth)acryl".

Examples of the phosphoric acid group-containing (meth)acrylic monomer include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenylhydrogen phosphate, 2-(meth)acryloyloxyethyl-2-bromoethylhydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and amine salts of these.

Examples of the phosphonic acid group-containing (meth)acrylic monomer include 2-(meth)acryloyloxyethylphenyl phosphonate, 5 - (meth)acryloyloxypentyl- 3 -phosphonopropionate, 6 - (meth)acryloyloxyhexyl- 3 -phosphonopropionate, 10-(meth)acryloyloxydecyl-3-phosphonopropionate, 6-(meth)acryloyloxyhexyl-phosphonoacetate, 10-(meth)acryloyloxydecyl-phosphonoacetate, and amine salts of these.

Examples of the pyrophosphoric acid group-containing (meth)acrylic monomer include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and amine salts of these.

Examples of the carboxylic acid group-containing (meth)acrylic monomer include:
(meth)acryhc acid, 4-(meth)acryloyloxyethoxycarbonylphthalic acid;
4-(meth)acryloyloxyethyl trimellitic acid, 4-(meth)acryloyloxybutyloxycarbonyl phthalic acid, 4-(meth)acryloyloxyhexyloxycarbonylphthalic acid, 4- (meth)acryloyloxyoctyloxycarbonyl phthalic acid, 4-(meth)acryloyloxydecyloxycarbonyl phthalic acid, and acid anhydrides of these; and
5-(meth)acryloylaminopentyl carboxylic acid, 6-(meth)acryloyloxy- 1,1-hexane dicarboxylic acid, 8-(meth)acryloyloxy-1,1-octane dicarboxylic acid, 10-(meth)acryloyloxy-1,1-decane dicarboxylic acid, 11-(meth)acryloyloxy-1,1-undecane dicarboxylic acid, and amine salts of these.

Examples of the sulfonic acid group-containing (meth)acryhc monomer include 2-(meth)acrylamide-2-methylpropane sulfonic acid, 2-sulfoethyl(meth)acrylate, and amine salts of these.

Preferred among these acidic group-containing radical polymerizable monomers (a) are (meth)acryhc monomers containing a phosphoric acid group, a pyrophosphoric acid group, or a carboxylic acid group, particularly phosphoric acid group-containing (meth)acryhc monomers, because these exhibit more desirable adhesion for dental restorations and tooth structure. More preferred are divalent phosphoric acid group-containing (meth)acrylic monomers having a C6 to C20 alkyl or alkylene group as a backbone within the molecule. Most preferred are divalent phosphoric acid group-containing (meth)acrylic monomers having a C8 to C12 alkylene group as a backbone within the molecule, for example, such as 10-methacryloyloxydecyl dihydrogen phosphate.

The acidic group-containing radical polymerizable monomer (a) may be added alone, or two or more thereof may be added in combination. The acidic group-containing radical polymerizable monomer (a) may fail to produce its effects when contained in excessively small amounts. When contained in excessively large amounts, excess remnants of acidic group-containing radical polymerizable monomer (a) on dental restorations and abutment teeth may impair the curability of the dental bonding agent applied in a later process, and the adhesive properties may decrease. The content of acidic group-containing radical polymerizable monomer (a) is not particularly limited, as long as the present invention can produce its effects. It is, however, preferable that the content of acidic group-containing radical polymerizable monomer (a) fall in a range of 0.1 to 30 parts by mass, more preferably 0.5 to 20 parts by mass, even more preferably 1.0 to 15 parts by mass, particularly preferably 2.0 to 8.0 parts by mass relative to total 100 parts by mass of the dental cleaner.

Aside from the acidic group-containing radical polymerizable monomer (a), a dental cleaner according to the present invention may comprise other polymerizable monomers, specifically a radical polymerizable monomer containing no acidic group. Examples of radical polymerizable monomers containing no acidic group include radical hydrophilic polymerizable monomers containing no acidic group, and hydrophobic polymerizable monomers containing no acidic group. Here, radical hydrophilic polymerizable monomers containing no acidic group means those having a solubility at 25°C of at least 10 mass% in water, preferably a solubility at 25°C of at least 30 mass% in water, more preferably a solubility that enables the monomer to dissolve in water in any desired fractions at 25°C. Hydrophobic polymerizable monomers containing no acidic group means those having a solubility at 25°C of less than 10 mass% in water. Examples of such hydrophobic polymerizable monomers containing no acidic group include crosslinkable polymerizable monomers such as aromatic bifunctional polymerizable monomers, aliphatic bifunctional polymerizable monomers, and tri- and higher-functional polymerizable monomers. In certain preferred embodiments, a dental cleaner of the present invention may comprise the acidic group-containing radical polymerizable monomer (a), water (b), and the organic amine compound (c) with essentially no radical polymerizable monomer containing no an acidic group. Here, essentially no radical polymerizable monomer containing no an acidic group means that the content of a radical polymerizable monomer containing no acidic group is less than 5.0 mass%, preferably less than 1.0 mass% relative to the total amount of the polymerizable monomers contained in the dental cleaner. In certain embodiments, a dental cleaner according to the present invention may comprise an acidic group-containing organic compound (a-2) having no polymerizable group. The acidic group-containing organic compound (a-2) having no polymerizable group may be the same compound representing the acidic group-containing organic compound (A-2) having no polymerizable group used for dental removers for temporary adhesives described below. The content of acidic group-containing organic compound (a-2) having no polymerizable group is also the same in dental removers for temporary adhesives.

The following describes water (b) used in the present invention.

Water (b) is a solvent for dissolving organic amine compound (c) and acidic group-containing radical polymerizable monomer (a). Water (b) also improves adhesive properties by promoting the acidic group-containing radical polymerizable monomer (a) to demineralize the tooth structure. It is required that water (b) be essentially free of impurities that have adverse effects on adhesive properties. Water (b) is preferably distilled water or ion-exchange water. The content of water (b) is preferably 40 to 99.8 parts by mass, more preferably 50 to 99 parts by mass, even more preferably 60 to 98 parts by mass relative to total 100 parts by mass of the dental cleaner.

A dental cleaner according to the present invention may comprise other solvents, in addition to water. By adding an additional solvent other than water, a more efficient cleaning effect may be expected by allowing for adjustments of the viscosity of the dental cleaner or by concentrating the active components. Examples of solvents other than water include organic solvents such as acetone and ethyl methyl ketone; and alcohols such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol. The content of solvents other than water (b) is not particularly limited, and is preferably at most 50 parts by mass, more preferably at most 40 parts by mass, even more preferably at most 20 parts by mass relative to total 100 parts by mass of the dental cleaner. The content of solvents other than water (b) is preferably at most 100 parts by mass, more preferably at most 85 parts by mass relative to 100 parts by mass of water (b). The content of solvents other than water (b) may be at most 60 parts by mass relative to 100 parts by mass of water (b).

The following describes the organic amine compound (c) used in the present invention.

The organic amine compound (c) provides an improved cleaning effect against contamination by protein-containing body fluids or substances originating from protein-containing body fluids, and improves the solubility of acidic group-containing radical polymerizable monomer (a) in water. This makes it possible to reduce generation of precipitates during refrigeration storage, providing uniform quality and imparting desirable bond strength and bond durability to the dental cleaner. By partly forming a salt through reaction with acidic group-containing radical polymerizable monomer (a), the organic amine compound (c) can produce surfactant action that further improves the cleaning effect against contamination by protein-containing body fluids. By containing organic amine compound (c), the dental cleaner can provide desirable bond strength and bond durability for dental restorations and tooth structure after cleaning. The organic amine compound (c) may contain a primary organic amine compound or a secondary organic amine compound. However, in view of reducing side reaction, it is preferable that the organic amine compound (c) contain a tertiary organic amine compound. The organic amine compound (c) may be an aliphatic compound or an aromatic compound. In certain embodiments, in order to enable a dental cleaner to provide more desirable bond strength and bond durability while ensuring uniform quality, the tertiary organic amine compound contained in the organic amine compound (c) is preferably an aliphatic compound having at least one hydroxyl group, or an aromatic compound having at least one hydroxyl group, more preferably an aliphatic compound having two or more hydroxyl groups, or an aromatic compound having two or more hydroxyl groups.

Examples of aliphatic compounds as tertiary organic amine compounds include N-methyl diethanolamine, N-ethyl diethanolamine, N-n-butyl diethanolamine, N-lauryl diethanolamine, 2-(dimethylamino)ethyl methacrylate, N-methyl diethanolamine dimethacrylate, N-ethyl diethanolamine dimethacrylate, triethanolamine monomethacrylate, triethanolamine dimethacrylate, triethanolamine trimethacrylate, triethanolamine, trimethylamine, triethylamine, and tributylamine. Triethanolamine and 2-(dimethylamino)ethyl methacrylate are more preferred for uniform quality and further improvement of bond strength and bond durability.

Examples of aromatic compounds as tertiary organic amine compounds include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, methyl 4-(N,N-dimethylamino)benzoate, ethyl 4-(N,N-dimethylamino)benzoate, butyl 4-(N,N-dimethylamino)benzoate, n-butoxyethyl 4-(N,N-dimethylamino)benzoate, 2-(methacryloyloxy)ethyl 4-(N,N-dimethylamino)benzoate, and 4-(N,N-dimethylamino)benzophenone. N,N-Bis(2-hydroxyethyl)-p-toluidine is more preferred for uniform quality and further improvement of bond strength and bond durability.

The organic amine compound (c) may be added alone, or two or more thereof may be added in combination. The organic amine compound (c) may fail to produce its effects when contained in excessively small amounts. Excessively large amounts of organic amine compound (c) pose a safety risk in the oral cavity because the increased basicity causes protein denaturation. The content of organic amine compound (c) is not particularly limited, as long as the present invention can produce its effects. However, in view of pH of the dental cleaner (a solution or a suspension), the content of organic amine compound (c) is preferably 0.1 to 20 parts by mass, more preferably 0.2 to 10 parts by mass, even more preferably 0.25 to 5 parts by mass relative to total 100 parts by mass of the dental cleaner.

Preferably, a dental cleaner of the present invention comprises a basic inorganic compound (d). The basic inorganic compound (d) is preferably at least one basic substance selected from a phosphate, a hydrogen phosphate, and a dihydrogen phosphate. The basic inorganic compound (d), even in small amounts, provides a cleaning effect against contamination by protein-containing body fluids and substances originating from protein-containing body fluids. The basic inorganic compound (d) partly forms a salt through a reaction with acidic group-containing radical polymerizable monomer (a), and can produce surfactant action that improves the cleaning effect against contamination by protein-containing body fluids. By containing basic inorganic compound (d), the dental cleaner provides desirable bond strength and bond durability for dental restorations and tooth structure after cleaning. Examples of the basic inorganic compound (d) include alkali metal salts of phosphoric acid, alkali metal salts of hydrogen phosphate, or alkali metal salts of dihydrogen phosphate, such as trisodium phosphate, disodium hydrogen phosphate, sodium dihydrogen phosphate, tripotassium phosphate, dipotassium phosphate, potassium dihydrogen phosphate; alkali-earth metal salts of phosphoric acid, alkali-earth metal salts of hydrogen phosphate, or alkali-earth metal salts of dihydrogen phosphate, such as tricalcium phosphate, calcium hydrogen phosphate, and calcium dihydrogen phosphate; and trimagnesium phosphate, and magnesium hydrogen phosphate. Preferably, the basic inorganic compound (d) is at least one selected from the group consisting of an alkali metal salt of phosphoric acid, an alkali metal salt of hydrogen phosphate, and an alkali metal salt of dihydrogen phosphate. More preferred are trisodium phosphate, disodium hydrogen phosphate, sodium dihydrogen phosphate, tripotassium phosphate, dipotassium phosphate, and potassium dihydrogen phosphate.

The basic inorganic compound (d) may be added alone, or two or more thereof may be added in combination. The basic inorganic compound (d) may fail to produce its effects when contained in excessively small amounts. Excessively large amounts of basic inorganic compound (d) pose a safety risk in the oral cavity because the increased basicity causes protein denaturation. When contained in excessively large amounts, the basic inorganic compound (d) itself may precipitate. This may impair ease of handling, and the dental cleaner may fail to produce a sufficient cleaning effect against protein-containing body fluids. The content of basic inorganic compound (d) is not particularly limited, as long as the present invention can produce its effects. However, in view of pH of the dental cleaner (a solution or a suspension), the content of basic inorganic compound (d) is preferably 0.1 to 20 parts by mass, more preferably 0.2 to 10 parts by mass, even more preferably 0.25 to 5 parts by mass relative to total 100 parts by mass of the dental cleaner.

A dental cleaner of the present invention has a pH of preferably less than 8.0. By satisfying this pH range, the dental cleaner can provide desirable bond strength and bond durability after cleaning while improving safety. The pH of a dental cleaner of the present invention is preferably at least 2.0 and less than 8.0, more preferably 2.5 to 7.5, even more preferably 3.0 to 6.5, particularly preferably 3.5 to 5.5. The pH can be measured using a known measurement device. The measurement device may be, for example, a LAQUAtwin manufactured by Horiba Ltd.

Preferably, a dental cleaner of the present invention comprises a gelatinizer (e). Examples of the gelatinizer (e) include a hydrophilic filler (e-1), a hydrophobic filler (e-2), and a polymeric thickener (e-3). The gelatinizer (e) may be used alone, or two or more thereof may be used in combination. In certain preferred embodiments of the present invention, the dental cleaner comprises a hydrophilic filler (e-1) and a hydrophobic filler (e-2) as gelatinizers (e). In other preferred embodiments, the dental cleaner comprises only a polymeric thickener (e-3) as gelatinizer (e). In other preferred embodiments, the dental cleaner comprises a hydrophilic filler (e-1), a hydrophobic filler (e-2), and a polymeric thickener (e-3). Preferably, the dental cleaner comprises a gelatinizer (e) combining all of hydrophilic filler (e-1), hydrophobic filler (e-2), and polymeric thickener (e-3). By using a hydrophilic filler (e-1), a hydrophobic filler (e-2), and a polymeric thickener (e-3) in combination, the dental cleaner can form a gel, allowing it to be more easily applied to a dental restoration or an abutment tooth such as a tooth surface. By using a hydrophilic filler (e-1), a hydrophobic filler (e-2), and a polymeric thickener (e-3) in combination, it is also possible to produce a cleaning effect against protein-containing body fluids or substances originating from protein-containing body fluids without interfering with the surfactant action.

By "hydrophilic filler (e-1)", it means a filler with a non-hydrophobized particle surface. For example, when the hydrophilic filler (e-1) is a particle having a surface silanol group such as silica, the non-hydrophobized particle surface is a surface with untreated silanol groups. By "hydrophobic filler (e-2)", it means a filler with a hydrophobized particle surface. For example, when the hydrophobic filler (e-2) is a particle having a surface silanol group such as silica, the hydrophobized particle surface is a surface with the silanol group hydrophobized with a substituent such as dimethylsilyl, trimethylsilyl, dimethylpolysiloxane, dimethylsiloxane, aminoalkylsilyl, methacrylsilyl, or alkylsilyl. The hydrophilic filler (e-1) is preferably a hydrophilic inorganic filler, more preferably hydrophilic fumed silica. The hydrophobic filler (e-2) is preferably a hydrophobic inorganic filler, preferably hydrophobic fumed silica. In the present specification, the average particle diameters of hydrophilic filler (e-1) and hydrophobic filler (e-2) are not particularly limited, and are preferably 0.0001 to 50 µm, more preferably 0.001 to 10 µm. In view of improving the spreadability of the dental cleaner, the average particle diameters of hydrophilic filler (e-1) and hydrophobic filler (e-2) are even more preferably 0.001 to 1.0 µm. The average particle diameters of hydrophilic filler (e-1) and hydrophobic filler (e-2) are average primary particle diameters, and can be determined by a laser diffraction scattering method or by observing particles with an electron microscope. Specifically, a laser diffraction scattering method is more convenient for particles of 0.1 µm or more, whereas electron microscopy is a more convenient method of particle diameter measurement for ultrafine particles of less than 0.1 µm. Here, 0.1 µm is a measured value by a laser diffraction scattering method. Specifically, for the measurement using a laser diffraction scattering method, for example, a laser diffraction particle size distribution analyzer (SALD-2300, manufactured by Shimadzu Corporation) may be used with a 0.2% sodium hexametaphosphate aqueous solution used as dispersion medium. For electron microscopy, a scanning electron microscope (for example, SU3800, S-4000, manufactured by HITACHI HIGH-TECHNOLOGIES CORPORATION) may be used. Specifically, in electron microscopy, for example, particles may be photographed with a scanning electron microscope, and the size of particles (at least 200 particles) observed in a unit field of the micrograph may be measured using image-analyzing particle-size-distribution measurement software (Macview; Mountech Co., Ltd.). Here, the particle diameter is determined as an arithmetic mean value of the maximum and minimum lengths of particles, and the average primary particle diameter is calculated from the number of particles and the particle diameter.

Examples of the hydrophilic filler (e-1) include hydrophilic fumed silica (fine silica particles manufactured by Nippon Aerosil Co., Ltd.), such as AEROSIL® 90, AEROSIL® 130, AEROSIL® 150, AEROSIL® 200, AEROSIL® 255, AEROSIL® 300, AEROSIL® 380, AEROSIL® OX50, AEROSIL® TT600, AEROSIL® 200 F, AEROSIL® 380 F, AEROSIL® 200 Pharma, and AEROSIL® 300 Pharma. Preferred is AEROSIL® 130.

Examples of the hydrophobic filler (e-2) include hydrophobic fumed silica (fine silica particles manufactured by Nippon Aerosil Co., Ltd.), such as AEROSIL® R972, AEROSIL® R974, AEROSIL® R104, AEROSIL® R106, AEROSIL® R202, AEROSIL® R208, AEROSIL® R805, AEROSIL® R812, AEROSIL® R812 S, AEROSIL® R816, AEROSIL® R7200, AEROSIL® R8200, AEROSIL® R9200, AEROSIL® R711, AEROSIL® R50, AEROSIL® NY50, AEROSIL® NY50 L, AEROSIL® NY200, AEROSIL® RY200 S, AEROSIL® RX50, AEROSIL® NY50, AEROSIL® RX, AEROSIL® RX00, AEROSIL® R504, AEROSIL® RNX90 S, AEROSIL® NX90 G, AEROSIL® RY300, AEROSIL® REA90, AEROSIL® REA200, AEROSIL® RY51, AEROSIL® NA50 Y, AEROSIL® RA200 HS, AEROSIL® NA50 H, AEROSIL® RA200 HS, AEROSIL® NA130 K, AEROSIL® NA200 Y, AEROSIL® NX130, AEROSIL® RY200 L, AEROSIL® R709, and AEROSIL® R976 S. Preferred is AEROSIL® R972.

The polymeric thickener (e-3) refers to a polymer added to the material to adjust viscosity, and that provides improved dispersibility and increased viscosity. The polymeric thickener (e-3) may be a natural substance or a synthetic substance. Example of natural substances include casein, methyl cellulose, carboxymethyl cellulose, and hydroxyethyl cellulose. Examples of synthetic substances include polyvinyl alcohol, sodium polyacrylate, polyethylene oxide, polyethylene glycol, and polyvinylpyrrolidone. The polymeric thickener (e-3) may be a commercially available product, for example, such as Macrogol 4000 or Macrogol 400 (polyethylene glycol thickeners manufactured by Maruishi Pharmaceutical Co., Ltd.), or polyvinylpyrrolidone (manufactured by IPS Corporation). The polymeric thickener (e-3) is preferably Macrogol 4000 (manufactured by Maruishi Pharmaceutical Co., Ltd.).

The content of gelatinizer (e) is not particularly limited, as long as the present invention can produce its effects. However, the content of gelatinizer (e) is preferably 0.50 to 40 parts by mass, more preferably 0.80 to 35 parts by mass, even more preferably 1 to 30 parts by mass, particularly preferably 4 to 25 parts by mass relative to total 100 parts by mass of the dental cleaner. The hydrophilic filler (e-1) is preferably hydrophilic fumed silica, and the hydrophobic filler (e-2) is preferably hydrophobic fumed silica. In view of inhibiting separation of the gel component and the liquid component in the dental cleaner, the mass ratio of the hydrophilic filler (e-1) and the hydrophobic filler (e-2) preferably ranges from 4:6 to 2:8, more preferably 4.3:5.7 to 2.2:7.8. In view of inhibiting separation of the gel component and the liquid component in the dental cleaner, the content of polymeric thickener (e-3) is preferably 1 to 25 parts by mass, more preferably 4 to 20 parts by mass, even more preferably 8 to 17 parts by mass relative to total 100 parts by mass of the dental cleaner.

A dental cleaner of the present invention may additionally comprise components such as a fluorine-ion releasing substance, a pH adjuster, a polymerization inhibitor (for example, dibutylhydroxytoluene (BHT), hydroquinone monomethyl ether (MEHQ)), a colorant, a fluorescent agent, and a fragrance, provided that such additional components do not interfere with the advantages of the present invention. It is also possible to add anti-microbial substances such as cetylpyridinium chloride, benzalkonium chloride, (meth) acryloyloxydodecylpyridinium bromide, (meth)acryloyloxyhexadecylpyridinium chloride, (meth)acryloyloxydecylammonium chloride, and triclosan.

A certain preferred embodiment (W-1) is, for example, a dental cleaner comprising an acidic group-containing radical polymerizable monomer (a), water (b), and an organic amine compound (c), wherein the acidic group-containing radical polymerizable monomer (a) contains at least one monomer selected from the group consisting of a phosphoric acid group-containing (meth)acryhc monomer, a pyrophosphoric acid group-containing (meth)acrylic monomer, and a carboxylic acid group-containing (meth)acrylic monomer, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-2) is, for example, a dental cleaner comprising an acidic group-containing radical polymerizable monomer (a), water (b), and an organic amine compound (c), wherein the acidic group-containing radical polymerizable monomer (a) contains at least one monomer selected from the group consisting of a phosphoric acid group-containing (meth)acryhc monomer, a pyrophosphoric acid group-containing (meth)acrylic monomer, and a carboxylic acid group-containing (meth)acrylic monomer, and the organic amine compound (c) contains a tertiary organic amine compound, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-3) is, for example, a dental cleaner comprising an acidic group-containing radical polymerizable monomer (a), water (b), and an organic amine compound (c), wherein the acidic group-containing radical polymerizable monomer (a) contains at least one monomer selected from the group consisting of a phosphoric acid group-containing (meth)acrylic monomer, a pyrophosphoric acid group-containing (meth)acrylic monomer, and a carboxylic acid group-containing (meth)acrylic monomer, the organic amine compound (c) contains a tertiary organic amine compound, and the tertiary organic amine compound contains an aliphatic compound, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-4) is, for example, a dental cleaner comprising an acidic group-containing radical polymerizable monomer (a), water (b), and an organic amine compound (c), wherein the acidic group-containing radical polymerizable monomer (a) is at least one selected from the group consisting of a phosphoric acid group-containing (meth) acrylic monomer, a pyrophosphoric acid group-containing (meth)acrylic monomer, and a carboxylic acid group-containing (meth)acrylic monomer, the organic amine compound (c) contains a tertiary organic amine compound, and the tertiary organic amine compound contains an aliphatic compound and an aromatic compound, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-5) is, for example, a dental cleaner comprising an acidic group-containing radical polymerizable monomer (a), water (b), and an organic amine compound (c), wherein the acidic group-containing radical polymerizable monomer (a) contains a phosphoric acid group-containing (meth)acrylic monomer, the organic amine compound (c) contains a tertiary organic amine compound, and the tertiary organic amine compound contains an aliphatic compound having two or more hydroxyl groups, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-6) is, for example, a dental cleaner comprising an acidic group-containing radical polymerizable monomer (a), water (b), and an organic amine compound (c), wherein the acidic group-containing radical polymerizable monomer (a) is at least one selected from the group consisting of a phosphoric acid group-containing (meth)acryhc monomer, a pyrophosphoric acid group-containing (meth)acrylic monomer, and a carboxylic acid group-containing (meth)acrylic monomer, the organic amine compound (c) contains a tertiary organic amine compound, and the tertiary organic amine compound contains an aliphatic compound and an aromatic compound, the aliphatic compound being an aliphatic compound having two or more hydroxyl groups, and the aromatic compound being an aromatic compound having two or more hydroxyl groups, and wherein the content of acidic group-containing radical polymerizable monomer (a) is 0.1 to 30 parts by mass, the content of water (b) is 40 to 99.8 parts by mass, and the content of organic amine compound (c) is 0.1 to 20 parts by mass relative to total 100 parts by mass of the dental cleaner. Another preferred embodiment (W-7) is, for example, a dental cleaner of any of the embodiments (W-1) to (W-6) above further comprising a basic inorganic compound (d), wherein the content of acidic group-containing radical polymerizable monomer (a) is 0.1 to 30 parts by mass, the content of water (b) is 40 to 99.8 parts by mass, and the total content of organic amine compound (c) and basic inorganic compound (d) is 0.1 to 20 parts by mass relative to total 100 parts by mass of the dental cleaner. Another preferred embodiment (W-8) is, for example, a dental cleaner of any of the embodiments (W-1) to (W-7) above further comprising a gelatinizer (e), wherein the content of acidic group-containing radical polymerizable monomer (a) is 0.1 to 30 parts by mass, the content of water (b) is 45 to 99.3 parts by mass, the content of organic amine compound (c) is 0.1 to 20 parts by mass, and the content of gelatinizer (e) is 0.50 to 40 parts by mass. In all of the preferred embodiments (W-1) to (W-8) above, the type and content of each component may be appropriately varied, and changes such as addition and deletion of any component may be made following the descriptions of the present specification.

A feature of a method for producing a dental cleaner of the present invention is that it includes a step of mixing an acidic group-containing radical polymerizable monomer (a), water (b), and an organic amine compound (c). The method and device used for mixing are not particularly limited, as long as the present invention can produce its effects. However, when the acidic group-containing radical polymerizable monomer (a) has low solubility in water (b), the method preferably mixes the acidic group-containing radical polymerizable monomer (a) after mixing the organic amine compound (c) into water (b), or mixes water (b) after mixing the organic amine compound (c) into the acidic group-containing radical polymerizable monomer (a). When producing a dental cleaner containing additional components (for example, gelatinizer (e)) other than the components (a) to (c), the additional components may be mixed simultaneously with the components (a) to (c), or the method may include a step of mixing the additional components, separately from the step of mixing the components (a) to (c).

Another embodiment of the present invention is, for example, nontherapeutic use of a dental cleaner comprising an acidic group-containing radical polymerizable monomer (a), water (b), and an organic amine compound (c) for removal of a dental temporary adhesive. The components of the dental cleaner are as described above. The dental cleaner also can be used for removal of a dental temporary adhesive. For removal of a dental temporary adhesive, the dental cleaner can be used for a nontherapeutic pretreatment performed before a restorative filling treatment that fills and lutes a final restoration. Yet another embodiment of the present invention is, for example, a dental cleaner comprising an acidic group-containing radical polymerizable monomer (a), water (b), and an organic amine compound (c) for a treatment that removes a dental temporary adhesive. The dental temporary adhesive will be described later.

Still another embodiment of the present invention is, for example, a dental remover for temporary adhesives (a dental remover composition for temporary adhesives) comprising an acidic group-containing compound (A), water (B), and a basic compound (C).

By reacting with basic compound (C) and forming a salt, the acidic group-containing compound (A) produces a desirable effect for removal of a temporary adhesive probably through 1) surfactant action producing a cleaning effect against organic compounds, for example, polyacrylic acid, contained in the temporary adhesive, and 2) water washing enabled by an acidic group adsorbing zinc ions derived from the zinc oxide representing a main component of the temporary adhesive adsorbed on a surface of a tooth structure.

The acidic group-containing compound (A) may be a known acidic group-containing compound. Preferred for use are, for example, an acidic group-containing polymerizable monomer (A-1), and an acidic group-containing organic compound (A-2) having no polymerizable group. The acidic group-containing polymerizable monomer (A-1) is more preferred because it can penetrate and bind to tooth structure through demineralization, and improve the adhesive properties of a luting agent for tooth structure through polymerization reaction with the polymerizable monomer component contained in the luting agent applied after the removal of a temporary adhesive.

The acidic group-containing polymerizable monomer (A-1) is a polymerizable monomer having at least one acidic group such as a phosphoric acid group, a phosphonic acid group, a pyrophosphoric acid group, a carboxylic acid group, and a sulfonic acid group, and at least one polymerizable group such as an acryloyl group, a methacryloyl group, an acrylamide group, and a methacrylamide group. The acidic group-containing polymerizable monomer (A-1) is preferably at least one selected from the group consisting of a phosphoric acid group-containing (meth)acrylic monomer, a pyrophosphoric acid group-containing (meth)acrylic monomer, and a carboxylic acid group-containing (meth)acryhc monomer. In view of adhesive properties for the enamel, the acidic group-containing polymerizable monomer (A-1) is preferably a monofunctional acidic group-containing (meth)acrylic monomer having any one of an acryloyl group, a methacryloyl group, an acrylamide group, and a methacrylamide group. Specific examples include the following.

Examples of the acidic group-containing polymerizable monomer (A-1) include the same compounds exemplified for the acidic group-containing radical polymerizable monomer (a) of the dental cleaner.

The acidic group-containing polymerizable monomer (A-1) is preferably a phosphoric acid group-, pyrophosphoric acid group- or carboxylic acid group-containing (meth)acrylic monomer, particularly a phosphoric acid group-containing (meth)acrylic monomer, because these develop desirable adhesive properties for the tooth structure. A longer carbon chain is preferred from the perspective of promoting micelle formation by the surfactant action considered in 1) above, whereas a shorter carbon chain is preferred when the steric hindrance of carbon chains for adsorption of zinc ions in 2) is taken into consideration. For efficient development of the both effects 1) and 2), the acidic group-containing polymerizable monomer (A-1) is more preferably a divalent phosphoric acid group-containing (meth)acryhc monomer having a C6 to C20 alkyl or alkylene group as a backbone within the molecule, most preferably a divalent phosphoric acid group-containing (meth)acryhc monomer having a C8 to C12 alkylene group as a backbone within the molecule, for example, such as 10-methacryloyloxydecyl dihydrogen phosphate.

The acidic group-containing organic compound (A-2) having no polymerizable group may be an organic compound having the same acidic group (e.g., a phosphoric acid group, a phosphonic acid group, a pyrophosphoric acid group, a carboxylic acid group, a sulfonic acid group) contained in the acidic group-containing polymerizable monomer (A-1). For the same reason discussed for the acidic group-containing polymerizable monomer (A-1), the acidic group-containing organic compound (A-2) having no polymerizable group is more preferably a divalent phosphoric acid group-containing compound having a C6 to C20 alkyl or alkylene group as a backbone within the molecule, even more preferably a divalent phosphoric acid group-containing compound having a C8 to C12 alkylene group as a backbone within the molecule.

The acidic group-containing compound (A) may be added alone, or two or more thereof may be added in combination. The acidic group-containing compound (A) may fail to produce its effects when contained in excessively small amounts. When contained in excessively large amounts, excess remnants of acidic group-containing compound (A) on cavities and abutment teeth may impair the curability of the dental bonding agent applied in a later process, and the adhesive properties may decrease. The content of acidic group-containing compound (A) is not particularly limited, as long as the present invention can produce its effects. However, the content of acidic group-containing compound (A) is preferably 0.1 to 30 parts by mass, more preferably 0.5 to 20 parts by mass, even more preferably 1.0 to 15 parts by mass, particularly preferably 2.0 to 8.0 parts by mass relative to total 100 parts by mass of the dental remover for temporary adhesives.

A dental remover for temporary adhesives according to the present invention may comprise a polymerizable monomer containing no acidic group. Examples of the polymerizable monomer containing no acidic group include a hydrophilic polymerizable monomer containing no acidic group, and a hydrophobic polymerizable monomer containing no acidic group. Here, hydrophilic polymerizable monomer containing no acidic group means those having a solubility at 25°C of at least 10 mass% in water, preferably a solubility at 25°C of at least 30 mass% in water, more preferably a solubility that enables the monomer to dissolve in water in any desired fractions at 25°C. Hydrophobic polymerizable monomer having no acidic group means those having a solubility at 25°C of less than 10 mass% in water. Examples of such hydrophobic polymerizable monomers having no acidic group include crosslinkable polymerizable monomers such as aromatic bifunctional polymerizable monomers, aliphatic bifunctional polymerizable monomers, and tri- and higher-functional polymerizable monomers. In certain preferred embodiments, the dental remover for temporary adhesives may comprise the acidic group-containing compound (A), water (B), and the basic compound (C) with essentially no polymerizable monomer containing no acidic group. Here, essentially no polymerizable monomer containing no acidic group means that the content of the polymerizable monomer containing no acidic group is less than 5.0 mass%, preferably less than1.0 mass%, more preferably less than 0.1 mass% relative to the total amount of the polymerizable monomers contained in the dental remover for temporary adhesives.

Water (B) is a solvent for dissolving basic compound (C) and acidic group-containing compound (A). Water (B) also improves adhesive properties by promoting the acidic group-containing compound (A) to demineralize the tooth structure. It is required that water (B) be essentially free of impurities that have adverse effects on adhesive properties. Water (B) is preferably distilled water or ion-exchange water. The content of water (B) is preferably 40 to 99.8 parts by mass, more preferably 45 to 99.3 parts by mass, even more preferably 50 to 98 parts by mass, particularly preferably 60 to 97 parts by mass relative to total 100 parts by mass of the dental remover for temporary adhesives.

In certain embodiments, a dental remover for temporary adhesives according to the present invention may additionally comprise a solvent other than water. By adding an additional solvent other than water, a more efficient cleaning effect may be expected by allowing for adjustments of the viscosity of the composition or by concentrating the active components. Examples of solvents other than water include organic solvents such as acetone and ethyl methyl ketone; and alcohols such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol. The content of solvents other than water (B) in such embodiments is not particularly limited, and is preferably at most 50 parts by mass, more preferably at most 40 parts by mass, even more preferably at most 20 parts by mass relative to total 100 parts by mass of the dental remover for temporary adhesives. The content of solvents other than water (B) is preferably at most 100 parts by mass, more preferably at most 85 parts by mass relative to 100 parts by mass of water (B). The content of solvents other than water (B) may be at most 60 parts by mass relative to 100 parts by mass of water (B).

The following describes the basic compound (C) used in the present invention. The basic compound (C) represents a basic inorganic compound (C-1) and an organic amine compound (C-2), as described below. The basic compound (C) may be added alone, or two or more thereof may be added in combination. For example, the basic inorganic compound (C-1) and the organic amine compound (C-2) may be used in combination. The content of basic compound (C) is not particularly limited, as long as the present invention can produce its effects. However, in view of pH of the dental remover for temporary adhesives (a solution or a suspension), the content of basic compound (C) is preferably 0.1 to 20 parts by mass, more preferably 0.2 to 10 parts by mass, even more preferably 0.25 to 8 parts by mass, particularly preferably 0.5 to 5 parts by mass relative to total 100 parts by mass of the dental remover for temporary adhesives.

Preferably, the basic inorganic compound (C-1) is at least one basic substance selected from a phosphate, sodium hydrogen phosphate, and sodium dihydrogen phosphate. The basic inorganic compound (C-1) partly forms a salt through a reaction with acidic group-containing compound (A), and can produce surfactant action that improves the cleaning effect against the organic components and zinc oxide components contained in the temporary adhesive. By containing basic inorganic compound (C-1), the dental remover for temporary adhesives provides desirable bond strength for tooth structure after cleaning. Examples of the basic inorganic compound (C-1) include the same compounds exemplified for the basic inorganic compound (d) of the dental cleaner.

The basic inorganic compound (C-1) may be added alone, or two or more thereof may be added in combination. The basic inorganic compound (C-1) may fail to produce its effects when contained in excessively small amounts. Excessively large amounts of basic inorganic compound (C-1) pose a safety risk in the oral cavity because the increased basicity causes protein denaturation. When contained in excessively large amounts, the basic inorganic compound (C-1) itself may precipitate. This may impair ease of handling, and the dental remover for temporary adhesives may fail to produce a sufficient cleaning effect against temporary adhesives. The content of basic inorganic compound (C-1) is not particularly limited, as long as the present invention can produce its effects. However, in view of pH of the dental remover for temporary adhesives (a solution or a suspension), the content of basic inorganic compound (C-1) is preferably 0.1 to 20 parts by mass, more preferably 0.2 to 10 parts by mass, even more preferably 0.25 to 8 parts by mass, particularly preferably 0.5 to 5 parts by mass relative to total 100 parts by mass of the dental remover for temporary adhesives.

The organic amine compound (C-2) improves the solubility of acidic group-containing compound (A) in water. By partly forming a salt through reaction with acidic group-containing compound (A), the organic amine compound (C-2) can produce surfactant action that further improves the cleaning effect against the organic components and zinc oxide components contained in the temporary adhesive. By containing organic amine compound (C-2), the dental remover for temporary adhesives can provide desirable bond strength durability for dental restorations and tooth structure after cleaning. The organic amine compound (C-2) may contain a primary organic amine compound or a secondary organic amine compound. However, in view of reducing side reaction, it is preferable that the organic amine compound (C-2) contain a tertiary organic amine compound. The tertiary organic amine compound may be an aliphatic compound or an aromatic compound.

Examples of aliphatic compounds and aromatic compounds as tertiary organic amine compounds include the same tertiary organic amine compounds exemplified for the organic amine compound (c) of the dental cleaner.

The organic amine compound (C-2) may be added alone, or two or more thereof may be added in combination. The organic amine compound (C-2) may fail to produce its effects when contained in excessively small amounts. Excessively large amounts of organic amine compound (C-2) pose a safety risk in the oral cavity because the increased basicity causes protein denaturation. The content of organic amine compound (C-2) is not particularly limited, as long as the present invention can produce its effects. However, in view of pH of the dental remover for temporary adhesives (a solution or a suspension), the content of organic amine compound (C-2) is preferably 0.1 to 20 parts by mass, more preferably 0.2 to 10 parts by mass, even more preferably 0.25 to 8 parts by mass, particularly preferably 0.5 to 5 parts by mass relative to total 100 parts by mass of the dental remover for temporary adhesives.

A dental remover for temporary adhesives of the present invention has a pH of preferably less than 8.0. By satisfying this pH range, the dental remover can provide desirable bond strength after cleaning while improving safety. The pH of a dental remover for temporary adhesives of the present invention is preferably at least 3.0 and less than 8.0, more preferably 3.5 to 7.5, even more preferably 4.5 to 7.4. The pH can be measured using a known measurement device. The measurement device may be, for example, a LAQUAtwin manufactured by Horiba Ltd.

Preferably, a dental remover for temporary adhesives of the present invention comprises a gelatinizer (D). Examples of the gelatinizer (D) include a hydrophilic filler (D-1), a hydrophobic filler (D-2), and a polymeric thickener (D-3). The gelatinizer (D) may be used alone, or two or more thereof may be used in combination. In certain preferred embodiments of the present invention, the dental remover for temporary adhesives comprises a hydrophilic filler (D-1) and a hydrophobic filler (D-2) as gelatinizers (D). In other preferred embodiments, the dental remover for temporary adhesives comprises only a polymeric thickener (D-3) as gelatinizer (D). In other preferred embodiments, the dental remover for temporary adhesives comprises a hydrophilic filler (D-1), a hydrophobic filler (D-2), and a polymeric thickener (D-3). Preferably, the dental remover for temporary adhesives comprises a gelatinizer (D) combining all of hydrophilic filler (D-1), hydrophobic filler (D-2), and polymeric thickener (D-3). By using a hydrophilic filler (D-1), a hydrophobic filler (D-2), and a polymeric thickener (D-3) in combination, the dental remover for temporary adhesives can form a gel, allowing it to be more easily applied to a dental restoration or an abutment tooth such as a tooth surface. By using a hydrophilic filler (D-1), a hydrophobic filler (D-2), and a polymeric thickener (D-3) in combination, it is also possible to produce a cleaning effect against the organic components and zinc oxide components contained in the temporary adhesive without interfering with the surfactant action.

The hydrophilic filler (D-1), hydrophobic filler (D-2), and polymeric thickener (D-3) used for a dental remover for temporary adhesives of the present invention share the same definitions used for the hydrophilic filler (e-1), hydrophobic filler (e-2), and polymeric thickener (e-3) of the dental cleaner. The hydrophilic filler (D-1), hydrophobic filler (D-2), and polymeric thickener (D-3) are also the same as their counterparts in the dental cleaner with regard to example compounds, average particle diameter, and method of measurement of average particle diameter.

The content of gelatinizer (D) is not particularly limited, as long as the present invention can produce its effects. However, the content of gelatinizer (D) is preferably 0.50 to 40 parts by mass, more preferably 0.80 to 35 parts by mass, even more preferably 1 to 30 parts by mass, particularly preferably 4 to 25 parts by mass relative to total 100 parts by mass of the dental remover for temporary adhesives. In certain embodiments, the hydrophilic filler (D-1) is preferably hydrophilic fumed silica, and the hydrophobic filler (D-2) is preferably hydrophobic fumed silica. In view of inhibiting separation of the gel component and the liquid component in the dental remover for temporary adhesives, the mass ratio of the hydrophilic filler (D-1) and the hydrophobic filler (D-2) preferably ranges from 4:6 to 2:8, more preferably 4.3:5.7 to 2.2:7.8. In view of inhibiting separation of the gel component and the liquid component in the dental remover for temporary adhesives, the content of polymeric thickener (D-3) is preferably 1 to 25 parts by mass, more preferably 4 to 20 parts by mass, particularly preferably 8 to 17 parts by mass relative to total 100 parts by mass of the dental remover for temporary adhesives.

A dental remover for temporary adhesives of the present invention may additionally comprise components such as a fluorine-ion releasing substance, a pH adjuster, a polymerization inhibitor (for example, dibutylhydroxytoluene (BHT), hydroquinone monomethyl ether (MEHQ)), a colorant, a fluorescent agent, and a fragrance, provided that such additional components do not interfere with the advantages of the present invention. A dental remover for temporary adhesives of the present invention may also comprise anti-microbial substances such as those exemplified for the dental cleaner.

A certain preferred embodiment (X-1) of the present invention is, for example, a dental remover for temporary adhesives comprising an acidic group-containing compound (A), water (B), and a basic compound (C), wherein the basic compound (C) contains a basic inorganic compound (C-1), and wherein the content of acidic group-containing compound (A) is 0.1 to 30 parts by mass, the content of water (B) is 40 to 99.8 parts by mass, and the content of basic inorganic compound (C-1) is 0.1 to 20 parts by mass relative to total 100 parts by mass of the dental remover for temporary adhesives. Another preferred embodiment (X-2) is, for example, a dental remover for temporary adhesives comprising an acidic group-containing compound (A), water (B), and a basic compound (C), wherein the basic compound (C) contains an organic amine compound (C-2), and wherein the content of acidic group-containing compound (A) is 0.1 to 30 parts by mass, the content of water (B) is 40 to 99.8 parts by mass, and the content of organic amine compound (C-2) is 0.1 to 20 parts by mass relative to total 100 parts by mass of the dental remover for temporary adhesives. Another preferred embodiment (X-3) is, for example, a dental remover for temporary adhesives comprising an acidic group-containing compound (A), water (B), and a basic compound (C), wherein the basic compound (C) contains a basic inorganic compound (C-1) and an organic amine compound (C-2), and wherein the content of acidic group-containing compound (A) is 0.1 to 30 parts by mass, the content of water (B) is 40 to 99.8 parts by mass, and the total content of basic inorganic compound (C-1) and organic amine compound (C-2) is 0.1 to 20 parts by mass relative to total 100 parts by mass of the dental remover for temporary adhesives. Another preferred embodiment (X-4) is, for example, a dental remover for temporary adhesives of any of the embodiments (X-1) to (X-3) above further comprising a gelatinizer (D), wherein the content of acidic group-containing compound (A) is 0.1 to 30 parts by mass, the content of water (B) is 45 to 99.3 parts by mass, the content of basic compound (C) is 0.1 to 20 parts by mass, and the content of gelatinizer (D) is 0.50 to 40 parts by mass. In all of the preferred embodiments (X-1) to (X-4) above, the type and content of each component may be appropriately varied, and changes such as addition and deletion of any component may be made following the descriptions of the present specification.

Examples of dental temporary adhesives that can be removed using a dental remover for temporary adhesives of the present invention include (1) Fletcher's cements (main components: zinc oxide and gum arabic), (2) eugenol-containing temporary adhesives (main components: zinc oxide and eugenol), (3) eugenol-free temporary adhesives (main components: zinc oxide, and aliphatic or aromatic compound), and (4) carboxylic acid-based temporary adhesives (main components: zinc oxide and acrylic acid polymer).

A feature of a method for producing a dental remover for temporary adhesives of the present invention is that it includes a step of mixing an acidic group-containing compound (A), water (B), and a basic compound (C). The method and device used for mixing are not particularly limited, as long as the present invention can produce its effects. However, when the acidic group-containing compound (A) has low solubility in water (B), the method preferably mixes the acidic group-containing compound (A) after mixing the basic compound (C) into water (B), or mixes water (B) after mixing the basic compound (C) into the acidic group-containing compound (A). When producing a dental remover for temporary adhesives containing additional components (for example, gelatinizer (D)) other than the components (A) to (C), the additional components may be mixed simultaneously with the components (A) to (C), or the method may include a step of mixing the additional components, separately from the step of mixing the components (A) to (C).

The present invention encompasses embodiments combining the foregoing features, provided that such combinations made in various forms within the technical idea of the present invention can produce the effects of the present invention.

### EXAMPLES

The following describes the present invention in greater detail by way of Examples. It should be noted that the present invention is in no way limited by the following Examples. The abbreviations used in Examples are as follows.

Acidic group-containing radical polymerizable monomer (a)
MDP: 10-Methacryloyloxydecyl dihydrogen phosphate Organic amine compound (c)
TTA: Triethanolamine
DEPT: N,N-Bis(2-hydroxyethyl)-p-toluidine
DMAEMA: 2-(Dimethylamino)ethyl methacrylate Gelatinizer (e)
(e-1): Hydrophilic fumed silica (a fine silica particle AEROSIL® 130 manufactured by Nippon Aerosil Co., Ltd.; average particle diameter: 16 nm)
(e-2): Hydrophobic fumed silica (a fine silica particle AEROSIL® R972 manufactured by Nippon Aerosil Co., Ltd.; average particle diameter: 16 nm)
(e-3): Polyvinylpyrrolidone (manufacturedby IPS Corporation), Macrogol 4000 (manufactured by Maruishi Pharmaceutical Co., Ltd.)

### Examples 1-1 to 1-9 and Comparative Examples 1-1 to 1-7

The dental cleaners of Examples and Comparative Examples were prepared as follows.

### Preparation of Dental Cleaner

For preparation of dental cleaners, the components in Tables 3 and 4 were mixed at ordinary temperature in the mass ratios shown in Tables 3 and 4. For use, the dental cleaners were individually charged into containers designed for Teethmate® Desensitizer, Liquid Formulation (manufactured by Kuraray Noritake Dental Inc.).

### Measurement of pH

The pH of each dental cleaner was measured using a pH meter (LAQUAtwin manufactured by Horiba Ltd.).

### Evaluation of Presence or Absence of Precipitate Formation during Refrigeration Storage

The dental cleaner was charged into a 440 mL container, and was visually checked for the presence or absence of precipitate formation during 1-week storage in a refrigerator at 4°C. The dental cleaners were determined as being "Satisfactory" when there was not any precipitate formation, and "Unsatisfactory" when precipitate formation was observed.

### Evaluation of Presence or Absence of Antiseptic Properties against Growth of Fungi and Other Microorganisms

The dental cleaners were evaluated for antiseptic properties by conducting a preservation efficacy test following the Preservatives-Effectiveness Test, General Information, provided in The Japanese Pharmacopoeia, 17th Edition. Amicrobial solution for testing was prepared by culturing the test microbial strains shown in Table 1 under the culture conditions of the Preservatives-Effectiveness Test. The test microbial solution was added to the dental cleaner, and the number of viable cells was counted after 2 weeks. Samples were determined as being "Satisfactory" when the viable cell count was less than 10 CPU for all strains relative to the initial viable cell count of 10⁵ CPU or more per milliliter. Samples were determined as being "Acceptable" when the viable cell count was less than 10⁵ CPU for all five strains, and 10 CPU or more and less than 10⁵ CPU for at least one of the strains, and "Unsatisfactory" when the viable cell count was 10⁵ CPU or more for at least one of the strains.

**[Table 1]**

| | | |
|---|---|---|
| Tested microbial strains | Bacteria | Escherichia coli |
| | | Pseudomonas aeruginosa |
| | | Staphylococcus aureus subsp.aureus |
| | Fungi | Candida albicans (yeast) |
| | | Aspergillus brasiliensis (mold) |

### Preparation of Artificial Saliva

The components in Table 2 were mixed at ordinary temperature in the proportions shown in Table 2 to prepare artificial salivae.

**[Table2]**

| | Molecular weight | Composition of artificial saliva (colorless transparent) | | |
|---|---|---|---|---|
| | | Amount used (g) | (mmol/L) | (mass%) |
| CaCl₂ | 110.98 | 0.0111 | 1.00 | 0.0111 |
| KH₂PO₄ | 136.09 | 0.0408 | 3.00 | 0.0408 |
| NaCl | 58.44 | 0.5844 | 100.00 | 0.5844 |
| NaOAc | 82.04 | 0.8204 | 100.00 | 0.8204 |
| NaN₃ | 65.01 | 0.0200 | 1.30 | 0.0200 |
| Casein¹⁾ | | 0.0100 | | 0.0100 |
| H₂O | | 100.0000 | | |

| | | | | |
|---|---|---|---|---|
| 1) Casein: FUJIFILM Wako Pure Chemical Corporation; Casein from milk | | | | |

### Bond Durability Test to Zirconia after Cleaning Procedure

A cylindrical zirconia sintered body measuring 12 mm in inner diameter and 5 mm in height (after firing at 1,500°C for 2 hours) fabricated from a zirconia disc for CAD/CAM system (manufactured by Kuraray Noritake Dental Inc. under the trade name KATANA® Zirconia HT) was ground with a #1000 silicon carbide paper (manufactured by Nihon Kenshi Co., Ltd.) to obtain a sample with an exposed flat surface. After being washed with a ultrasonic cleaner (manufactured by Yamato Scientific Co., Ltd.) for 5 minutes, the sample was dried by blowing air and removing water on sample surface. The zirconia sintered body was then immersed in artificial saliva for 1 minute, and was dried by blowing air. Thereafter, the dental cleaner prepared was applied to the flat surface of the zirconia sintered body, and the surface was cleaned by being rubbed for 10 seconds, followed by rinsing with water. After drying the surface by blowing air, an adhesive tape, about 150 µm thick and having a 5 mm circular hole, was attached to the surface to provide a surface having a defined bonding area for adherends.

A dental resin cement (manufactured by Kuraray Noritake Dental Inc. under the trade name SA Cement Plus Automix®) was placed on the surface prepared for adherends, using a mixing tip. A cylindrical stainless steel rod (measuring 7 mm in diameter and 2.5 cm in length) was then bonded at its one end face (circular end face) to the surface, and was left to stand for 60 minutes. The sample was immersed in distilled water after removing the excess dental resin cement from around the cylindrical stainless steel rod. The sample in distilled water was left to stand first in a thermostatic chamber for 24 hours at the maintained temperature of 37°C, and then in a thermostatic chamber for 7 days at the maintained temperature of 70°C to obtain a sample for adhesion testing. A total of five samples were prepared for adhesion testing.

The five samples for adhesion testing were measured for tensile bond strength by using a universal testing machine (manufactured by Shimadzu Corporation) with the crosshead speed set at 2 mm/min. The values presented in Tables 3 and 4 are mean values. The variation (SD) of tensile bond strengths was calculated using the mean value of tensile bond strengths as bond durability. The bond durability is preferably 16 MPa or more, more preferably 18 MPa or more, even more preferably 20 MPa or more. The variation (SD) of tensile bond strengths is preferably 5 or less, more preferably 4 or less, even more preferably 2.5 or less.

### Test for cleaning effect against Zirconia

A cylindrical zirconia sintered body measuring 12 mm in inner diameter and 5 mm in height (after firing at 1,500°C for 2 hours) fabricated from a zirconia disc for CAD/CAM system (manufactured by Kuraray Noritake Dental Inc. under the trade name KATANA® Zirconia HT) was ground with a lapping film sheet (manufactured by The 3M Company; grain size: 1 micron) to obtain a sample with an exposed flat surface. After being washed with a ultrasonic cleaner (manufactured by Yamato Scientific Co., Ltd.) for 5 minutes, the sample was dried by blowing air against water on sample surface. The sample was then immersed in artificial saliva for 1 minute, and was dried by blowing air. Thereafter, the dental cleaner was applied to the flat surface of the sample, and the surface was cleaned by being rubbed for 10 seconds, followed by rinsing with water. Here, the dental cleaner was used after being stored in a refrigerator at 4°C for 1 week, and was used for cleaning without removing precipitates even when the dental cleaner formed precipitates during storage. After drying the surface by blowing air, the sample was observed for cleaning effect, using a scanning electron microscope (SU3500 manufactured by HITACHI HIGH-TECHNOLOGIES CORPORATION) at 1,000 times magnification. The results were evaluated using the following criteria (n = 1).
Satisfactory: No deposits were present after cleaning
Unsatisfactory: Deposits were present after cleaning

**[Table 3]**

| | | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 |
|---|---|---|---|---|---|---|---|---|---|---|---|
| Acidic group-containing radical polymerizable monomer (a) | | MDP | 5.0 | 5.0 | 4.0 | 4.0 | 4.5 | 3.0 | 5.0 | 4.0 | 4.0 |
| Water (b) | | Water | 92.0 | 84.0 | 70.0 | 70.0 | 76.0 | 60.0 | 93.0 | 78.0 | 78.0 |
| Organic amine compound (c) | | TTA | | | 1.5 | 1.0 | 1.5 | 1.0 | 2.0 | 1.0 | 1.5 |
| | | DEPT | | | 0.5 | 0.5 | 0.5 | | | 0.5 | 0.5 |
| | | DMAEMA | 2.0 | 2.0 | | | | | | | |
| Basic inorganic compound (d) | | Na₂HPO₄ | 1.0 | 1.0 | | 0.5 | 0.5 | | | 0.5 | |
| Organic Solvent | | Ethanol | | | | | | | | | |
| | | Acetone | | | | | | | | | |
| Gelatinizer (e) | Hydrophilic filler (e-1) | Hydrophilic fumed silica AEROSIL 130 | | 3.0 | 3.0 | 3.0 | | 6.0 | | | |
| | Hydrophobic filler (e-2) | Hydrophobic fumed silica AEROSIL R972 | | 5.0 | 6.0 | 6.0 | | 10.0 | | | |
| | Polymeric thickener (e-3) | Polyvinylpyrrolidone | | | | | | 16.0 | | | |
| | | Macrogol 4000 | | | 15.0 | 15.0 | 17.0 | 4.0 | | 16.0 | 16.0 |
| Total [paris by mass] | | | 100.0 | 100.0 | 100.0 | 100.0 | 100.0 | 100.0 | 100.0 | 100.0 | 100.0 |
| pH | | | 6.4 | 6.5 | 4.5 | 4.7 | 4.6 | 4.2 | 4.0 | 4.7 | 4.5 |
| Presence or Absence of precipitate formation during refrigeration storage | | | Satisfactory | Satisfactory | Satisfactory | Satisfactory | Satisfactory | Satisfactory | Satisfactory | Satisfactory | Satisfactory |
| Presence or absence of antiseptic properties against microbial growth (incl. fungi) | | | | | | | Satisfactory | | Satisfactory | Satisfactory | Satisfactory |
| Bond durability Tensile bond strength to zirconia after cleaning (MPa) (70°C, 7 d) | | | 20.2 | 20.0 | 25.3 | 25.0 | 25.6 | 21.0 | 22.6 | 26.0 | 26.2 |
| Variation of tensile bond strength (SD) | | | 1.4 | 1.0 | 1.3 | 1.2 | 1.1 | 1.5 | 1.6 | 1.2 | 1.0 |
| Cleaning effect against zirconia | | | Satisfactory | Satisfactory | Satisfactory | Satisfactory | Satisfactory | Satisfactory | Satisfactory | Satisfactory | Satisfactory |

**[Table 4]**

| | | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 |
|---|---|---|---|---|---|---|---|---|---|
| Acidic group-containing radical polymerizable monomer (a) | | MDP | 1.0 | 5.0 | 5.0 | 10.0 | 8.0 | 3.0 | 5.0 |
| Water (b) | | Water | 98.0 | 90.0 | 94.0 | 69.0 | 63.0 | 76.0 | 74.0 |
| Organic amine compound (c) | | TTA | | | | | | | |
| | | DEPT | | | | | | | |
| | | DMAEMA | | | | | | | |
| Basic inorganic compound (d) | | Na₂HPO₄ | 1.0 | 5.0 | 1.0 | 1.0 | 1.0 | 1.0 | 1.0 |
| Organic Solvent | | Ethanol | | | | 20.0 | 8.0 | | 20.0 |
| | | Acetone | | | | | | 20.0 | |
| Gelatinizer (e) | Hydrophilic filler (e-1) | Hydrophilic fumed silica AEROSIL 130 | | | | | 5.0 | | |
| | Hydrophobic filler (e-2) | Hydrophobic fumed silica AEROSIL R972 | | | | | | | |
| | Polymeric thickener (e-3) | Polyvinylpyrrolidone | | | | | | | |
| | | Macrogol 4000 | | | | | 15.0 | | |
| Total [paris by mass] | | | 100.0 | 100.0 | 100.0 | 100.0 | 100.0 | 100.0 | 100.0 |
| pH | | | 6.4 | 6.4 | 3.4 | 2.7 | 3.0 | 4.4 | 3.2 |
| Presence or Absence of precipitate formation during refrigeration storage | | | Unsatisfactory | Unsatisfactory | Unsatisfactory | Unsatisfactory | Unsatisfactory | Unsatisfactory | Unsatisfactory |
| Presence or absence of antiseptic properties against microbial growth (incl. fungi) | | | | | | | | | |
| Bond durability Tensile bond strength to zirconia after cleaning (MPa) (70°C, 7 d) | | | 12.8 | 14.6 | 15.0 | 13.6 | 14.2 | 13.0 | 14.0 |
| Variation of tensile bond strength (SD) | | | 7.1 | 7.0 | 6.3 | 6.9 | 7.9 | 6.0 | 6.1 |
| Cleaning effect against zirconia | | | Unsatisfactory | Unsatisfactory | Unsatisfactory | Unsatisfactory | Unsatisfactory | Unsatisfactory | Unsatisfactory |

As can be seen from Table 3, the dental cleaners according to the present invention provided a desirable bond durability of 20 MPa or more in the examination of bond durability performed with a dental cement applied to cleaned zirconia, showing that the dental cleaners according to the present invention exhibit a desirable cleaning effect against contamination by protein-containing body fluids. There was also no observable formation of precipitates during storage under refrigerated conditions. Scanning electron microscope observation of zirconia surfaces cleaned with the dental cleaners stored in a refrigerator at 4°C for 1 week revealed that the dental cleaners according to the present invention have a desirable cleaning effect. In contrast, as shown in Table 4, the cleaning effect against contamination by protein-containing body fluids was poor, and it was not possible to obtain the desired bond durability in Comparative Examples. The bond durability of the dental cement against cleaned zirconia was 15 MPa or less, and precipitate formation was observed after storage under refrigerated conditions.

As shown in Table 3, the dental cleaners of Examples 1-5, 1-7, 1-8, and 1-9 exhibited antiseptic properties, inhibiting growth of viable microorganisms. Accordingly, the evaluation results were satisfactory for Examples 1-5, 1-7, 1-8, and 1-9. The organic amine compound (c) was shown to exhibit antiseptic properties by itself or with one or more other organic amine compounds (c). When added, the basic inorganic compound (d) was also shown to exhibit antiseptic properties.

The compounds used for the dental removers for temporary adhesives of Examples and Comparative Examples are listed below with the abbreviations used.
Acidic group-containing compound (A)
   Acidic group-containing polymerizable monomer (A-1)
   MDP: 10-Methacryloyloxydecyl dihydrogen phosphate
Basic compound (C)
   Basic inorganic compound (C-1)
   Na₂HPO₄: Disodium hydrogen phosphate
Organic amine compound (C-2)
   TTA: Triethanolamine
   DEPT:N,N-Biₛ(2-hydroxyethyl)-p-toluidine
   DMAEMA: 2-(Dimethylamino)ethyl methacrylate
Gelatinizer (D)
   (D-1): Hydrophilic fumed silica (a fine silica particle AEROSIL® 130 manufactured by Nippon Aerosil Co., Ltd.)
   (D-2): Hydrophobic fumed silica (a fine silica particle AEROSIL® R972 manufactured by Nippon Aerosil Co., Ltd)
   (D-3): Macrogol 4000 (manufactured by Maruishi Pharmaceutical Co., Ltd.)

### Examples 2-1 to 2-5 and Comparative Examples 2-1 to 2-3

The dental removers for temporary adhesives of Examples and Comparative Examples were prepared as follows.

### Preparation of Dental Remover for Temporary Adhesives

For preparation of dental removers for temporary adhesives, the components in Table 5 were mixed at ordinary temperature in the mass ratios shown in Table 5. For use, the dental removers for temporary adhesives were individually charged into containers designed for Teethmate® Desensitizer, Liquid Formulation (manufactured by Kuraray Noritake Dental Inc.).

### Temporary Adhesive

A High-Bond Temporary Cement, Soft (a carboxylic acid-based temporary adhesive containing zinc oxide, a sodium salt of acrylic acid-tricarboxylic acid copolymer, and an HY agent; manufactured by Shofu Inc.) was used as temporary adhesive.

### Measurement of pH

The pH of each dental remover for temporary adhesives was measured using a pH meter (LAQUAtwin manufactured by Horiba Ltd.).

### Test for cleaning effect against Temporary Adhesive

Labial surfaces of bovine mandibular incisors were ground with a (#80) silicon carbide paper (manufactured by Nihon Kenshi Co., Ltd.) under running water to obtain samples with an exposed flat dentin surface. The flat surface of sample was then ground with a #1000 silicon carbide paper (manufactured by Nihon Kenshi Co., Ltd.) under running water. The resulting sample was washed with a ultrasonic cleaner (manufactured by Yamato Scientific Co., Ltd.) for 5 minutes, and was dried by blowing air against water on sample surface. A High-Bond Temporary Cement, Soft, kneaded following IFU (instruction manual), was applied as a temporary adhesive to the flat surface of the dentin sample. The bovine tooth (dentin) sample with the applied temporary adhesive was then put on a Kimtowel placed in a container after being wetted with distilled water. After sealing the container, the adhesive was cured at 37°C for 30 minutes, and the container was kept in 37°C water for 1 week. The sample was taken out after this 1 week of storage, and the temporary adhesive was removed. After washing the sample with water and subsequent air blowing, the dental remover for temporary adhesives was applied to the area of sample that had the temporary adhesive. The sample was then cleaned by rubbing the dental remover for 10 seconds, and the surface was rinsed with water. After drying the surface by blowing air, the sample was observed for cleaning effect, using a scanning electron microscope (SU3500 manufactured by HITACHI HIGH-TECHNOLOGIES CORPORATION) at 1,000 times magnification. The results were evaluated using the following criteria (n = 1).
Satisfactory: No deposits were present after cleaning
Unsatisfactory: Deposits were present after cleaning

### Tensile Adhesion Test for Bovine Teeth after Removal of Temporary Adhesive

Labial surfaces of bovine mandibular incisors were ground with a (#80) silicon carbide paper (manufactured by Nihon Kenshi Co., Ltd.) under running water to obtain samples with an exposed flat dentin surface. The flat surface of sample was then ground with a #1000 silicon carbide paper (manufactured by Nihon Kenshi Co., Ltd.) under running water. The resulting sample was washed with a ultrasonic cleaner (manufactured by Yamato Scientific Co., Ltd.) for 5 minutes, and was dried by blowing air against water on sample surface. A High-Bond Temporary Cement, Soft, kneaded following IFU (instruction manual), was applied as a temporary adhesive to the flat surface of the dentin sample. The bovine tooth (dentin) sample with the applied temporary adhesive was then put on a Kimtowel placed in a container after being wetted with distilled water. After sealing the container, the adhesive was cured at 37°C for 30 minutes, and the container was kept in 37°C water for 1 week. The sample was taken out after this 1 week of storage, and the temporary adhesive was removed. After washing the sample with water and subsequent air blowing, the dental remover for temporary adhesives was applied to the area of sample that had the temporary adhesive. The sample was then cleaned by rubbing the dental remover for 10 seconds, and the surface was rinsed with water. After drying by air blowing, an adhesive tape, about 150 µm thick and having a 3-mm circular hole, was attached to the surface to provide a surface having a defined bonding area for adherends.

A dental resin cement (manufactured by Kuraray Noritake Dental Inc. under the trade name SA Cement Plus Automix®) was placed on the surface prepared for adherends, using a mixing tip. A cylindrical stainless steel rod (measuring 7 mm in diameter and 2.5 cm in length) was then bonded at its one end face (circular end face) to the surface, and was left to stand for 60 minutes. The sample was immersed in distilled water after removing the excess dental resin cement from around the cylindrical stainless steel rod. The sample in distilled water was left to stand in a thermostatic chamber for 24 hours at the maintained temperature of 37°C to obtain a sample for adhesion testing. A total of five samples were prepared for adhesion testing.

The five samples for adhesion testing were measured for tensile bond strength by using a universal testing machine (manufactured by Shimadzu Corporation) with the crosshead speed set at 2 mm/min. The values presented in Table 5 are mean values. The tensile bond strength is preferably 7 MPa or more, more preferably 7.5 MPa or more. The samples had a tensile bond strength of 8 MPa in a tensile adhesion test performed in the same fashion without applying the temporary adhesive before bonding. The samples had a tensile bond strength of 2 MPa when cleaned without the dental remover for temporary adhesives after the removal of the temporary adhesive.

**[Table5]**

| | | | Ex. 2-1 | Ex. 2-2 | Ex. 2-3 | Ex. 2-4 | Ex. 2-5 | Com. Ex. 2-1 | Com. Ex. 2-2 | Com. Ex. 2-3 |
|---|---|---|---|---|---|---|---|---|---|---|
| Composition of dental remover for temporary adhesive used for cleaning | Acidic group-containing polymerizable monomer (A-1) | MDP | 5.0 | 5.0 | 4.0 | 4.5 | 3.0 | 5.0 | | |
| | Water (B) | Water | 92.0 | 84.0 | 70.0 | 76.0 | 94.0 | | 97.0 | 97.0 |
| | Basic inorganic compound (C-1) | Na₂HPO₄ | 1.0 | 1.0 | 0.5 | 0.5 | | | | 3.0 |
| | Organic amine compound (C-2) | TTA | | | 1.0 | 1.5 | 3.0 | | 3.0 | |
| | | DEPT | | | 0.5 | 0.5 | | | | |
| | | DMAEMA | 2.0 | 2.0 | | | | | | |
| | Organic Solvent | Ethanol | | | | | | 95.0 | | |
| | Gelatinizer (D) | (D-1) Hydrophilic fumed silica AEROSIL 130 | | 3.0 | 3.0 | | | | | |
| | | (D-2) Hydrophobic fumed silica AEROSIL R972 | | 5.0 | 6.0 | | | | | |
| | | (D-3) Macrogol 4000 | | | 15.0 | 17.0 | | | | |
| | Total [paris by mass] | | 100.0 | 100.0 | 100.0 | 100.0 | 100.0 | 100.0 | 100.0 | 100.0 |
| pH | | | 6.4 | 6.5 | 4.7 | 4.6 | 7.4 | 1.5 | 9.1 | 10.1 |
| Cleaning effect against temporary adhesive | | | Satisfactory | Satisfactory | Satisfactory | Satisfactory | Satisfactory | Unsatisfactory | Unsatisfactory | Unsatisfactory |
| Tensile bond strength to bovine tooth (dentin) after removal of temporary adhesive [MPa] (37°C, 1 d) | | | 7.8 | 7.0 | 7.8 | 7.6 | 7.7 | 2.8 | 3.4 | 3.0 |

As can be seen from Table 5, the dental removers for temporary adhesives according to the present invention provided a desirable tensile bond strength of 7 MPa or more in the examination of tensile bond strength performed with a dental resin cement applied to a bovine tooth (dentin) after the removal procedure, showing that the dental removers for temporary adhesives according to the present invention exhibit a desirable cleaning effect against the organic components and zinc oxide contained in the temporary adhesive. Scanning electron microscope observation of bovine teeth surfaces cleaned with the dental removers for temporary adhesives revealed that the dental removers for temporary adhesives according to the present invention have a desirable cleaning effect against the temporary adhesive. In contrast, the tensile bond strength was about 3 MPa in all of Comparative Examples. The inferior tensile bond strength after the removal of temporary adhesive is probably due to the poor cleaning effect against the organic components and zinc oxide contained in the temporary adhesive.

### INDUSTRIAL APPLICABILITY

A dental cleaner according to the present invention can be used for applications such as cleaning of dental restorations and abutment teeth. A dental remover for temporary adhesives according to the present invention provides desirable cleanliness against temporary adhesives-a material used temporarily only for a short duration of time-while ensuring desirable bond strength after cleaning.

## Claims

1. A dental cleaner comprising an acidic group-containing radical polymerizable monomer (a), water (b), and an organic amine compound (c).

2. The dental cleaner according to claim 1, wherein the dental cleaner has a pH of less than 8.0.

3. The dental cleaner according to claim 1 or 2, wherein the acidic group-containing radical polymerizable monomer (a) comprises at least one monomer selected from the group consisting of a phosphoric acid group-containing (meth)acrylic monomer, a pyrophosphoric acid group-containing (meth)acrylic monomer, and a carboxylic acid group-containing (meth)acrylic monomer.

4. The dental cleaner according to any one of claims 1 to 3, wherein the organic amine compound (c) comprises a tertiary organic amine compound.

5. The dental cleaner according to claim 4, wherein the tertiary organic amine compound comprises an aliphatic compound.

6. The dental cleaner according to claim 5, wherein the aliphatic compound is triethanolamine and/or 2-(dimethylamino)ethyl methacrylate.

7. The dental cleaner according to any one of claims 4 to 6, wherein the tertiary organic amine compound comprises an aromatic compound.

8. The dental cleaner according to any one of claims 1 to 7, wherein the dental cleaner further comprises a basic inorganic compound (d).

9. The dental cleaner according to claim 8, wherein the basic inorganic compound (d) is at least one basic substance selected from the group consisting of a phosphate, a hydrogen phosphate, and a dihydrogen phosphate.

10. The dental cleaner according to claim 8 or 9, wherein the basic inorganic compound (d) is at least one selected from the group consisting of an alkali metal salt of phosphoric acid, an alkali metal salt of hydrogen phosphate, and an alkali metal salt of dihydrogen phosphate.

11. The dental cleaner according to any one of claims 1 to 10, wherein the dental cleaner further comprises at least one gelatinizer (e) selected from the group consisting of a hydrophilic filler (e-1), a hydrophobic filler (e-2), and a polymeric thickener (e-3).

12. The dental cleaner according to claim 11, wherein the gelatinizer (e) is a hydrophilic filler (e-1) and a hydrophobic filler (e-2).

13. The dental cleaner according to claim 12, wherein the hydrophilic filler (e-1) is hydrophilic fumed silica, and the hydrophobic filler (e-2) is hydrophobic fumed silica.

14. The dental cleaner according to claim 12 or 13, wherein the hydrophilic filler (e-1) and the hydrophobic filler (e-2) have a mass ratio of 4:6 to 2:8.

15. A method for producing a dental cleaner, comprising mixing an acidic group-containing radical polymerizable monomer (a), water (b), and an organic amine compound (c).

16. Nontherapeutic use of a dental cleaner comprising an acidic group-containing radical polymerizable monomer (a), water (b), and an organic amine compound (c) for removal of a dental temporary adhesive.

17. A dental remover for temporary adhesives, comprising an acidic group-containing compound (A), water (B), and a basic compound (C).

18. The dental remover for temporary adhesives according to claim 17, wherein the dental remover has a pH of less than 8.0.

19. The dental remover for temporary adhesives according to claims 17 or 18, wherein the acidic group-containing compound (A) is an acidic group-containing polymerizable monomer (A-1).

20. The dental remover for temporary adhesives according to claim 19, wherein the acidic group-containing polymerizable monomer (A-1) is at least one selected from the group consisting of a phosphoric acid group-containing (meth)acrylic monomer, a pyrophosphoric acid group-containing (meth)acrylic monomer, and a carboxylic acid group-containing (meth)acrylic monomer.

21. The dental remover for temporary adhesives according to any one of claims 17 to 20, wherein the basic compound (C) is at least one selected from the group consisting of a basic inorganic compound (C-1) and an organic amine compound (C-2).

22. The dental remover for temporary adhesives according to claim 21, wherein the basic inorganic compound (C-1) is at least one basic substance selected from the group consisting of a phosphate, a hydrogen phosphate, and a dihydrogen phosphate.

23. The dental remover for temporary adhesives according to claim 21 or 22, wherein the basic inorganic compound (C-1) is at least one selected from the group consisting of an alkali metal salt of phosphoric acid, an alkali metal salt of hydrogen phosphate, and an alkali metal salt of dihydrogen phosphate.

24. The dental remover for temporary adhesives according to claim 21, wherein the organic amine compound (C-2) comprises a tertiary organic amine compound.

25. The dental remover for temporary adhesives according to any one of claims 17 to 24, wherein the dental remover further comprises a gelatinizer (D).

26. The dental remover for temporary adhesives according to claim 25, wherein the gelatinizer (D) comprises a hydrophilic filler (D-1) and a hydrophobic filler (D-2).

27. The dental remover for temporary adhesives according to claim 26, wherein the hydrophilic filler (D-1) is hydrophilic fumed silica, and the hydrophobic filler (D-2) is hydrophobic fumed silica.

28. The dental remover for temporary adhesives according to claim 26 or 27, wherein the hydrophilic filler (D-1) and the hydrophobic filler (D-2) have a mass ratio of 4:6 to 2:8.

29. The dental remover for temporary adhesives according to any one of claims 25 to 28, wherein the gelatinizer (D) comprises a polymeric thickener (D-3).

30. A method for producing a dental remover for temporary adhesives of claim 17, comprising mixing an acidic group-containing compound (A), water (B), and a basic compound (C).
